# EUROPÄISCHE PATENTSCHRIFT

(11) **EP 4 591 828 B1**
(45) Veröffentlichungstag und Bekanntmachung des Hinweises auf die Patenterteilung: **11.02.2026**
(21) Anmeldenummer: 24218402.6
(22) Anmeldetag: 09.12.2024
(51) Int. Cl.: A61F 2/18, A61F 2/00

(54) **EINSEITIGES SEPTUM-IMPLANTAT MIT SPLINT**
SINGLE SIDED SEPTAL IMPLANT WITH SPLINT
IMPLANT SEPTALE MONOFACE AYANT UNE SPLLINCT

(30) Priorität: 24.01.2024 DE 102024101972
(43) Veröffentlichungstag der Anmeldung: 30.07.2025
(73) Patentinhaber: Heinz Kurz GmbH, 72144 Dusslingen (DE)
(72) Erfinder: SANDER, Alexander, 79822 Titisee-Neustadt (DE); STIEGELE, Thomas, 72585 Riederich (DE); MERTENS, Matthias, 24568 Kaltenkirchen (DE); À WENGEN, Daniel Felix, 4103 Bottmingen (CH)
(74) Vertreter: Kohler Schmid Möbus Patentanwälte

(56) Entgegenhaltungen:
- EP-A1- 1 475 056
- DE-B3- 102022 120 193
- US-A1- 2014 039 619
- US-A1- 2023 030 410
- US-B2- 9 949 823

## Beschreibung

Die Erfindung betrifft ein rhinologisches Implantat zur Begradigung der Nasenscheidewand, das auf einer Außenfläche des Septums der menschlichen Nase in der linken und/oder rechten Seite der Nasenhöhle befestigbar ist und im implantierten Zustand auf dieser Außenfläche des Septums vollflächig anliegt, wobei das Implantat aus einem flachen, mit mehreren durchgängigen Perforationen versehenen Zuschnitt aufgebaut ist.

Ein derartiges rhinologisches Implantat zur Begradigung der Nasenscheidewand geht hervor aus dem generischen Stand der Technik DE 10 2022 120 193 B3 ≈ EP 23 180 536.7 (= Referenz [1]) oder aus DE 10 2012 107 123 B4 ≈ EP 2 692 313 B1 ≈ US 9 895 252 B2 (= Referenz [2]) der Anmelderin.

### Hintergrund der Erfindung

Generell sind Implantate zur Septum-Begradigung beispielsweise beschrieben auf der Internetseite vom 19.01.2024 https://bess.eu/rhinologie/septumschienen/septumschienen (= Referenz [3])

Die Einpflanzung eines Implantats, d. h. eines in der Regel körperfremden Gewebsstücks oder Stoffes, in den menschlichen Körper ist in der Medizintechnik ein lange bekanntes Verfahren, das in vielen Variationen zur Behebung funktioneller Störungen verschiedener Körperteile und/oder psychischer Beeinträchtigungen vorgenommen wird.

Bekanntlich besteht der knorpelige Anteil der menschlichen Nase aus der Nasenscheidewand (=Septum), dem Dreiecksknorpel und dem Flügelknorpel. Die vorliegende Erfindung beschäftigt sich mit der Begradigung des Septums. In vielen Fällen der funktionellen, aber auch der kosmetischen Nasenchirurgie ist es das Ziel, das Septum zu begradigen, um den Luftstrom innerhalb des Nasenverlaufs zu verbessern oder schlicht die Nase kosmetisch zu verschönern. Meist geht dieser Vorgang noch mit der Veränderung anderer Strukturen einher. Derzeit werden zur Begradigung des Septums unterschiedliche Naht- oder Knorpeltransplantattechniken verwendet.

Im Folgenden sollen zunächst die Unterschiede zwischen einer Septumplastik und der Columella-Strut-Technik erläutert werden:
Die Septumplastik zielt ausschließlich darauf ab, das Septum zu begradigen und damit die Luftzufuhr über die Nase zu optimieren. Es handelt sich also um rein funktionelle Chirurgie. Hierzu gibt es eine Reihe von chirurgischen Techniken, die über Nähte und Transplantate zum Ziel führen sollen.

Die Begradigung eines deviierten Septums kann eine der größten Herausforderungen bei einer Rhinoplastik sein. Unterschiedliche und im Wesentlichen von der Erfahrung und dem Geschick des Operateurs abhängige Verfahren kommen hierfür in Betracht.

Disartikulationstechniken bis hin zu gezielten Knorpelinzisionen können mehr oder weniger eine Rolle spielen. In einer Sache ist sich die Fachwelt hier einig, dass eine Septumbegradigung gerade im mittleren Gewölbe eine schwierige Angelegenheit und keineswegs eine einfache Operation ist.

Im Gegensatz zur Septumbegradigung zielt die Columella-Strut-Technik darauf ab, die Nasenspitze aus kosmetischen Gründen heraus zu erhöhen. Dabei wird ein Steg zwischen die beiden Enden der beiden Flügelknorpel geschoben, um die Spitze der Nase zu erhöhen. Das Transplantat/ Implantat wird in eine Tasche zwischen den medialen Schenkeln der Flügelknorpel über die Spina nasalis anterior aufgestellt und zwischen den medialen Schenkeln mit durchgreifenden Nähten befestigt.

Dies ist beispielsweise beschrieben in der US 2012/0078367 A1 (= Referenz [4]). Hier wird von einem Implantat gesprochen, das biologisch abgebaut wird und somit nicht für den Langzeitverbleib vorgesehen ist. Die Zielregion ist das untere Drittel der Nase, hauptsächlich die Region um die Nasenspitze. Zwar ist hier auch die Rede von einer Anbindung des Implantates an das Septum, allerdings nicht zur Begradigung desselben, sondern zur Verlängerung, falls durch äußere Einwirkung oder aus kosmetischer Sicht das Septum verkürzt ist. Die gebogenen Teile des beschriebenen Implantats sind in ihrer Längenausdehnung höchstens etwa 20mm lang, d.h. sie reichen, wenn man sie auf die Basis der Spina Nasalis setzt und die beiden Schenkel der Flügelknorpel aufnimmt, gerade noch ans Ende des Septums. Letzteres wird damit also niemals begradigt. Bereits der Sitz des Implantats im implantierten Zustand wäre für Septumbegradigung völlig ungeeignet.

Das Implantat nach Referenz [4] ist daher keinesfalls zur Begradigung der Nasenscheidewand geeignet, und ist auch nicht beiderseits des Septums der menschlichen Nase auf der jeweiligen Außenfläche des Septums in der jeweils linken und rechten Seite der Nasenhöhle befestigbar. Vielmehr handelt es sich bei dem Implantat gemäß Referenz [4] um ein "Columella Strut", nicht jedoch um eine gattungsgemäße Septumplastik entsprechend der vorliegenden Erfindung zur Begradigung der Nasenscheidewand. Ein Hilfsmittel zur Begradigung des Septums thematisiert und erwähnt Referenz [4] überhaupt nicht.

Auch in der WO 2008/153263 A1 (= Referenz [5]) werden ausschließlich Techniken beschrieben, die zum Ziel haben, die Nasenspitze anzuheben. So ist dort zwar ebenfalls vom Septum die Rede. Beschrieben wird etwa die Technik, dass das Implantat an der oberen Grenze des Septumknorpels angebracht werden muss. Allerdings geschieht dies auch hier wieder lediglich zur Stabilisierung des Nasenspitzenimplantats.

Die bekannten Implantations-Systeme nach Referenz [4] und nach Referenz [5] versuchen also, das Ende des Septums zur Stabilisierung oder zur Verlängerung zu verwenden. Keines der beiden Systeme zielt jedoch darauf ab oder wäre geeignet dazu, das Septum dauerhaft zu begradigen.

Implantate zur Spreizung der Nasenflügel sind etwa beschrieben in US 6 322 590 B1 (= Referenz [6]) oder in EP 1 475 056 B1 (= Referenz [7]) oder in DE 10 2006 023 058 B3 (= Referenz [8]). Auch diese Implantations-Systeme sind keinesfalls zur Septum-Begradigung ausgebildet oder geeignet.

Zur Begradigung des Septums hingegen sind Techniken mit PDS-Folien bekannt (etwa beschrieben in: HNO.1999 Jun;47 (6):546-50 (= Referenz [9])), welche zurechtgeschnitten und auf das Septum aufgenäht werden. Die hauchdünnen Folien-Implantate resorbieren nach 10 bis 25 Wochen im Wesentlichen rückstandsfrei.

Stabilere -jedoch ebenfalls nicht dauerhafte- Implantate zur Septumbegradigung stellen die Septumschienen nach Reuter aus Silikon dar, wie sie auf der eingangs zitierten Internetseite der Firma Bess beschrieben sind. Diese Implantate dienen zur Schienung des Septums und zur Minimierung des Adhäsionsrisikos zwischen Septum und lateraler Nasenwand nach Rhinoplastik. Sie sind zum leichteren Einführen und Entfernen geschlitzt und weisen vorgestanzte Löcher zur Nahtbefestigung auf. Als Material wird Fluorplastik angegeben. Die Schienen müssen allerdings nach ein paar Wochen wieder aus der Nase herausgenommen werden, da Silikon als nicht langzeit-stabil gilt und potentiell zu Infektionen führt.

CN 21 538 4901 U (= Referenz [10]) offenbart eine Nasenkorrekturstütze für die plastische Chirurgie, die einen Columella-nasi-Stützabschnitt, einen Nasenseptum-Verlängerungsabschnitt, Blättchen des Columella-nasi-Stützabschnitts, Blättchen des Nasenseptum-Verlängerungsabschnitts, eine Nasenspitzecke und eine Columella nasi umfasst mit einer Klemmnut am Stützende, einer Klemmnut am Kopfende der Nasenscheidewandverlängerung und einer U-förmigen Nut.

US 11 241 306 B2 (= Referenz [11]) beschreibt Nasenimplantate mit einem planaren Profil, welches teilweise offene Räume aufweist. Ein solches Nasenimplantat kann kompressibel entlang einer oder mehrerer Dimensionen sein, wie etwa der Breite oder der Länge des planaren Profils.

Gegenüber dem in den Referenzen [3] bis [11] gezeigten Stand der Technik vermeidet der eingangs zitierte Stand der Technik gemäß Referenz [2] die Nachteile der Septumschienen nach Reuter und das Implantat liegt nach der Operation dauerhaft eng an der Nasenscheidewand an.

Die Grundidee von Referenz [2] ist es, das Septum einfach mit einer speziell geformten und dadurch mechanisch dauerhaft stabilen Struktur zu begradigen. Dabei ist es wichtig, dass das Implantat gemäß Referenz [2] geometrisch so ausgebildet ist, dass der gesamte Bereich des Septums abgedeckt wird. Dies wird dadurch erreicht, dass das Implantat die freie Unterkante des Septums umgreift. Weiter sind beide Seiten des Implantats so perforiert, dass das Implantat einfach und sicher an beiden Seiten des Septums fixiert werden kann. Die Dicke und damit einhergehende Steifigkeit des Implantats ist insofern wichtig, als dass der Septumknorpel sich langfristig der Implantatform angleichen sollte und nicht umgekehrt.

Die beiden Seitenabschnitte des Implantats gemäß Referenz [2] weisen jeweils einen ersten Teilabschnitt auf, der sich unmittelbar an den zentralen Rückenabschnitt anschließt und im Wesentlichen parallel zu diesem verläuft, und die beiden Seitenabschnitte weisen zu ihren freien Enden hin jeweils einen zweiten Teilabschnitt auf, der sich an den jeweiligen ersten Teilabschnitt anschließt und gegenüber diesem abgewinkelt verläuft. Durch diese einfachen Maßnahmen wird die mechanische Langzeit-Stabilität des Implantats erhöht. Außerdem wird der flächige feste Sitz des Implantats an den beiden Seitenflächen der Nasenscheidewand verbessert.

Eine vergleichbare geometrische Vorschrift im Stand der Technik gemäß den Referenzen [3] bis [11] liegt nicht im Funktionsumfang der dort beschriebenen Columella-Strut-Technik sowie der Nasenflügel-Spreizung und bedient daher ein ganz anderes, eigenständiges Versorgungsfeld.

Der auch bereits eingangs zitierte Stand der Technik gemäß Referenz [1] schließlich stellt eine Verbesserung gegenüber dem rhinologischen Implantat gemäß Referenz [2] vor: Hier wird vorgeschlagen, dass sich an einem dem jeweiligen zweiten Teilabschnitt gegenüberliegenden Ende des jeweiligen ersten Teilabschnitts gemäß Referenz [2] jeweils ein dritter Teilabschnitt anschließt und gegenüber dem jeweiligen ersten Teilabschnitt ebenfalls abgewinkelt verläuft. Dadurch wird eine noch bessere mechanische Langzeit-Stabilisierung des Septums ermöglicht und der flächige feste Sitz des Implantats an den beiden Seitenflächen der Nasenscheidewand noch weiter verbessert. Insbesondere wird eine sehr gleichmäßige Verteilung der Anlagekräfte bei dem auf dem Septum angebrachten Implantat erreicht.

Bei den meisten aktuell erhältlichen Septum-Implantaten zur Korrektur von Septum-Deviationen oder Perforationen des Septums werden die Implantat-Strukturen mit Knorpel gestützt. Demgegenüber haben die Implantate gemäß den Referenzen [1] oder [2] vielerlei Vorteile:
Es muss kein Knorpel aus anderen Bereichen (normalerweise Rippe) entnommen werden. Dadurch verringert sich die Operationszeit und es besteht weniger Infektionsgefahr. Weiter ist bei Implantaten gemäß den Referenzen [1] oder [2] zu erwarten, dass eine Stabilisierung aus Metall deutlich länger die gewünschte Begradigung leistet, als dies mit verformbarem Knochen wie bei einigen Implantaten nach dem Stand der Technik möglich wäre. Schließlich kann davon ausgegangen werden, dass ein Implantat gemäß den Referenzen [1] oder [2] deutlich dünner aufträgt als vergleichbar stabiler Knorpel, was sich positiv auf den gewünschten Luftdurchsatz auswirkt.

### Aufgabe der Erfindung

Der vorliegenden Erfindung liegt demgegenüber die Aufgabe zugrunde, mit unaufwändigen technischen Mitteln ein rhinologisches Implantat der gattungsbildenden Art gemäß Referenz [1] oder [2] mit den eingangs definierten Merkmalen dahingehend zu modifizieren, dass die Vorrichtung nicht über den Nasenrücken-seitigen Teil des Septums gesetzt und auf beiden Seiten der Nasenscheidewand angebracht werden muss, sondern eine einseitige Anwendung ermöglicht wird. Außerdem soll der Fertigungsaufwand wesentlich verringert und ein erheblicher Anteil an Material und Gewicht eingespart werden.

### Kurze Beschreibung der Erfindung

Diese relativ komplexe Aufgabe wird auf überraschend einfach und kostengünstig zu realisierende Weise dadurch gelöst, dass das Implantat eine ebene Form aufweist und im implantierten Zustand flach auf lediglich einer einzigen der beiden Außenflächen des Septums vollflächig anliegt; dass das Implantat einen dreiecksförmigen Grundkörper aufweist, bei welchem von einer Dreiecksspitze ausgehend zwei Katheten-Kanten unter einem Dreieckswinkel φ gegeneinander angeordnet sind, geradlinig von der Dreiecksspitze wegführen und beiderseits jeweils in Richtung auf eine der Dreiecksspitze gegenüberliegende Hypotenusen-Kante verlaufen; und dass der dreiecksförmige Grundkörper an den beiden Katheten-Kanten durch zwei jeweils von der Dreiecksspitze weg über die Hypotenusen-Kante hinausgreifende Seitenabschnitte verlängert ist, wobei die Außen-Kanten der beiden Seitenabschnitte jeweils in Flucht mit ihrer jeweiligen Katheten-Kante verlaufen.

Wie auch schon die generischen rhinologischen Implantate dient das erfindungsgemäße Implantat insbesondere zur Begradigung, Schienung, Stützung sowie zum Aufbau und zur Wiederherstellung der knorpligen humanen Nasenscheidewand (=Septum).

Die neue Grundidee der Erfindung ist dabei, ein lediglich einseitig einsetzbares Implantat vorzustellen, welches nur auf eine Seite des Septum-Knorpels aufgenäht wird. Dadurch wird die Implantations-OP wesentlich vereinfacht, weil zum Einsetzen des Implantats eben nur noch auf einer Seite des Septums die Nasenschleimhaut vom Knorpel der Scheidewand abgehoben werden muss, um das flache und starre Implantat dann einzuschieben.

Zwar können theoretisch auch zwei Implantate auf beiden Seiten des Septums eingesetzt werden, um etwa eine besonders hohe mechanische Stabilität zu erreichen oder um asymmetrische Deformationen der Nasen-Scheidewand feinfühliger und besser korrigieren zu können. Auch in solchen Fällen wird aber immerhin durch das Fehlen eines Nasenrücken-Abschnitts am erfindungsgemäßen Implantat die OP deutlich unaufwändiger und damit weniger belastend für den Patienten.

Die Materialeinsparung bei der Herstellung des erfindungsgemäßen Implantats gegenüber dem oben diskutierten generischen Stand der Technik ist ebenfalls erheblich. Aufgrund des Weglassens des bisher obligatorischen Nasenrücken-Abschnitts sowie des gegenüberliegenden weiteren Seitenteils werden 60-70% geringere Materialmengen benötigt, was natürlich auch zu einer großen Gewichtseinsparung beim erfindungsgemäßen Implantat führt.

Schließlich wird auch das Fertigungsverfahren ganz wesentlich vereinfacht: Das Implantat wird stets im ursprünglichen flachen, ungebogenen Zustand des Zuschnitts belassen. Ein späteres Aufrichten in eine Raumform nach dem Ausstanzen des flachen Zuschnitts (wie bei den generischen Implantaten gemäß Referenz [1] oder [2] stets zwingend erforderlich) entfällt nun vollständig, was wiederum zu einer deutlichen Arbeitsersparnis bei der Herstellung des erfindungsgemäßen Implantats beiträgt.

Damit wird trotz des geringeren Fertigungsaufwands eine besonders gute geometrische Anpassung des Implantats an die aktuelle, möglicherweise auch stark deformierte Ausbildung des jeweiligen Patienten-Septums und eine besonders gute Anlage des Implantats an einer der Außenflächen desselben erreicht. Insbesondere entstehen nach der Operation keine Hohlräume zwischen dem Implantat und dem Septum. Vielmehr liegt das flache Implantat nach der OP über seine gesamte dem Septum zugewandte Oberfläche eng an diesem an, was auch einen besonders guten mechanischen Halt des Implantats am Septum bewirkt.

Durch vorkonfektionierte erfindungsgemäße Implantate kann der Eingriff besonders gut reproduzierbar, standardisiert und planbar durchgeführt werden. Wenn eine gewisse Anzahl unterschiedlicher Größen und auch Formen des erfindungsgemäßen Implantats vorgehalten wird, kann der Operateur besonders genau auf die individuellen geometrischen Verhältnisse am jeweiligen Patienten-Septum optimal eingehen.

### Bevorzugte Ausführungsformen der Erfindung

Ganz besonders bevorzugt sind Ausführungsformen des erfindungsgemäßen Implantats, welche sich dadurch auszeichnen, dass die Dreiecksspitze sowie vorzugsweise auch alle weiteren, zumindest die außenliegenden, Ecken des Implantats abgerundet ausgeführt sind. Damit lässt sich einerseits während der Implantation ein Hängenbleiben oder gar Aufspießen sicher verhindern, andererseits wird dadurch auch im implantierten Zustand bei etwaigen post-operativen Bewegungen des Implantats relativ zu seiner Umgebung eine Verletzungsgefahr ausgeschlossen.

In einer weiteren bevorzugten Ausführungsform der Erfindung weisen die beiden Seitenabschnitte des Implantats jeweils eine zu ihrer Außen-Kante mit Abstand parallel verlaufende Innen-Kante auf.

Die Seitenabschnitte sind in diesem Fall geometrisch als im Wesentlichen rechteckige "Zungen" ausgeführt, die sich auf beiden Seiten des dreiecksförmigen Grundkörpers längs der beiden Katheten-Kanten von der Dreiecksspitze weg über die Hypotenusen-Kante hinaus erstrecken und fertigungstechnisch besonders einfach und präzise herstellbar sind.

In einer Klasse von besonders bevorzugten Ausführungsformen ist das Implantat symmetrisch zu einer von der Dreiecksspitze rechtwinklig auf die Hypotenusen-Kante verlaufenden Höhen-Achse, die das Implantat lateral in zwei spiegelbildliche Hälften aufteilt, aufgebaut.

Diese symmetrische Formgebung ist fertigungstechnisch besonders einfach und präzise zu realisieren.

Alternativ dazu zeichnet sich eine weitere Klasse von Ausführungsformen der Erfindung dadurch aus, dass das Implantat asymmetrisch aufgebaut ist, wobei ein erster Seitenabschnitt eine größere Längenausdehnung von der Dreiecksspitze des dreiecksförmigen Grundkörpers weg aufweist als der zweite Seitenabschnitt.

Auf diese Weise kann in einem Sortiment mit unterschiedlichen Formen und Größen auf individuelle Besonderheiten und ungewöhnliche Deformationen des jeweiligen Patienten-Septums, die sich mit einer symmetrischen Form des Implantats nicht optimal behandeln ließen, besondere Rücksicht genommen werden.

Wie bereits oben beschrieben, weist das erfindungsgemäße Implantat eine Vielzahl von Perforationen auf. Damit wird einerseits die Masse des Implantats reduziert und mithin dessen Gewicht verringert, andererseits der Anteil von körperfremdem Material, das durch das Implantat in den menschlichen Körper eingebracht wird, so weit wie möglich minimiert. Außerdem fördern die Perforationen das Verwachsen des Implantats mit dem Gewebe.

Die Perforationen sind vorzugsweise sowohl am dreiecksförmigen Grundkörper als auch an den beiden Seitenabschnitten des Implantats vorhanden und können zum Beispiel als kreisrunde Löcher oder als Langlöcher ausgebildet sein.

Weiter dienen diese Perforationen auch einer sicheren Fixierung am Dreiecksknorpel des Septums durch Annähen des Implantats an den Knorpel und/oder den knöchernen Nasenboden insbesondere an den unteren vorderen Nasensporn, mittels einer OP-Naht. Damit kann zudem sichergestellt werden, dass das Knorpelgewebe und die Knorpelhaut des Septums ausreichend mit Nährstoffen versorgt wird.

Die Herstellung einer dauer-haltbaren Befestigung des Implantats durch Aufnähen am Knorpel ist allerdings in der Regel zeitintensiv und manchmal auch aufgrund der räumlichen Verhältnisse etwas kompliziert. Vorteilhafte Varianten des erfindungsgemäßen Implantats zeichnen sich daher auch dadurch aus, dass in den dreiecksförmigen Grundkörper und/oder in die Seitenabschnitte ein oder mehrere Spikes eingearbeitet sind. Letztere verkrallen sich bei der Implantation im Septum-Knorpel und sorgen so für einen exzellenten und äußerst dauerhaften Halt des Implantats. Diese Art der Befestigung kann freilich auch mit anderen der oben beschriebenen Befestigungsarten kombiniert werden, um einen besonders gesicherten Sitz des Implantats am Septum zu garantieren.

Bei weiteren vorteilhaften Ausführungsformen der Erfindung können die Perforationen des Implantats zusammen einen größeren Flächeninhalt aufweisen als die die Perforationen umgebenden, insbesondere steg-förmig ausgebildeten, festen Teilabschnitte des Implantats.

Damit lassen sich die Masse und mithin das Gewicht des erfindungsgemäßen Implantats bei dennoch hoher mechanischer Stabilität deutlich minimieren. Auch wird hierdurch eine ausreichende Versorgung des Knorpelgewebes am Septum mit Nährstoffen sichergestellt.

Vorzugsweise kann bei Varianten der Erfindung zumindest ein Teil der Perforationen polyederförmig oder wabenförmig ausgebildet sein.

Dies ermöglicht eine hohe Stabilität auch bei geringem Materialeinsatz.

Zumindest ein Teil der Perforationen kann aber auch schlitzförmig, vorzugsweise durch untereinander parallel verlaufende längliche Schlitze, ausgebildet sein.

Damit wird unter anderem die Nahtführung für den implantierenden Chirurgen erleichtert.

Besonders bevorzugt sind Ausführungsformen des erfindungsgemäßen Implantats, bei welchen sämtliche Kanten des Implantats, zumindest alle außenliegenden Kanten, abgerundet ausgeführt sind.

Damit wird eine mögliche Verletzungsgefahr minimiert, das Einwachsen in das menschliche Gewebe wird erheblich erleichtert und die Bedeckung durch die Weichteile der Knorpelhaut sowie der Nasenschleimhaut wird merklich verbessert.

In der Praxis bewähren sich Ausführungsformen der Erfindung, bei welchen die Dicke des flachen Zuschnitts für das Implantat zwischen 0,2mm und 1,2mm, vorzugsweise etwa 0,8mm, beträgt.

Das erfindungsgemäße Implantat kann bis maximal die Ausdehnung eines gesamten menschlichen Nasenscheidewand-Knorpels einnehmen und weist eine größte Längenausdehnung in der Zuschnitt-Ebene zwischen 2mm und 7mm, vorzugsweise etwa 5mm auf.

Für die allermeisten denkbaren Anwendungsfälle des erfindungsgemäßen Implantats eignen sich Ausführungsformen, bei denen der Dreieckswinkel φ an der Dreiecksspitze des dreiecksförmigen Grundkörpers zwischen 60° und 120°, vorzugsweise etwa 100°, beträgt.

Diese Formgebung dient der Verstärkung des Implantats und vermindert damit das Eintreten einer Verbiegung durch äußere Kräfte im Sinne eines Knotenblechs.

Für einige Anwendungen kann es vorteilhaft sein, wenn das Implantat ganz oder teilweise aus Metall gefertigt ist. Als Material für chirurgische und orthopädische Implantate, die dauerhaft im Körper verbleiben sollen, sind Metalle und deren Legierungen prädestiniert, weil sie neben einer sehr guten Biokompatibilität hohe Dauerfestigkeit und Elastizität aufweisen. Trotz einer relativ geringen Dichte besitzen Implantate aus solchen Materialien wie reines Titan oder Titanverbindungen ausgezeichnete mechanische Eigenschaften mit einer langen Lebensdauer. Ebenfalls eine gute Eignung für die genannten Zwecke besitzen nichtmagnetisierbare Metalle beziehungsweise Legierungen, mit denen auch MRT-Sicherheitsaspekte berücksichtigt werden können.

Bevorzugt sind Ausführungsformen, bei welchen das Implantat aus einem Werkstoff mit Memory-Effekt und/oder superelastischen Eigenschaften, vorzugsweise aus Nitinol, aufgebaut ist, so dass beispielsweise durch geeignete thermische Behandlung des Implantats optimale Federeigenschaften relativ zum Septum eingebracht werden können.

Für andere Anwendungen kann es vorteilhaft sein, wenn das Implantat ganz oder teilweise aus Kunststoff gefertigt ist, insbesondere aus Polymer-Material, vorzugsweise aus einem Material, welches eine ähnliche Flexibilität wie das menschliche Septum aufweist, aber über genügend Steifigkeiten verfügt, um eine gekrümmte Nasenscheidewand langfristig zu begradigen, und ohne eine Durchtrennung des Septums erforderlich zu machen, um eine Schwächung herbeizuführen.

Zusätzlich zur guten Biokompatibilität des verwendeten Materials selbst kann das Implantat auch einen besonderen, körperverträglichen und/oder keimabweisenden Überzug aufweisen.

Zur Erzielung einer fein abgestimmten Form des Implantats kann dieses zweckmäßigerweise mittels 3D-Drucktechnik und/oder mittels Lasertechnik hergestellt sein.

Entsprechend den individuellen Anforderungen kann das erfindungsgemäße Implantat aber auch in spanender Technik, insbesondere mittels Frästechnik und/oder mittels Schleiftechnik, vorzugsweise Gleitschleifen, Magnetgleitschleifen oder SatellitenFliehkraftschleifen, und/oder mittels Poliertechnik, vorzugsweise Elektropolieren, oder in Ätztechnik hergestellt werden.

Bei weiteren vorteilhaften Ausführungsformen der Erfindung schließlich ist das Implantat im Spritzguss nach dem Micro Injection Moulding (=MIM) Verfahren gefertigt, welches beispielsweise aus der WO 00/06327 A2 (= Referenz [12]) an sich bekannt ist. Damit kann eine äußerst preisgünstige Herstellung auch sehr großer Stückzahlen bei gleichbleibender Maßgenauigkeit erreicht werden, während die herkömmlichen Implantate in der Regel wie etwa Schmuckwaren handangefertigt werden und daher einerseits relativ teuer in der Herstellung sind, andererseits in der Maßgenauigkeit individuell variieren können.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen.

### Detaillierte Beschreibung der Erfindung anhand der Zeichnung

In der schematischen Zeichnung sind Ausführungsbeispiele der Erfindung dargestellt, welche in der nachfolgenden Beschreibung näher erläutert werden.

Es zeigen:
- Fig.1: eine schematische Draufsicht in stark vergrößerter Darstellung senkrecht von oben auf die Zuschnitts-Ebene einer symmetrischen Ausführungsform des erfindungsgemäßen Implantats mit exakt spiegelbildlich zueinander ausgebildeten Seitenabschnitten in großer L-Größe;
- Fig. 2a: die Ausführungsform von Fig. 1, jedoch in etwas kleinerer (gegenüber der Realität aber immer noch stark vergrößerter) Darstellung;
- Fig. 2b: die Ausführungsform von Fig. 2a, jedoch mit Blickrichtung von der Seite in der Zuschnitts-Ebene;
- Fig. 2c: die Ausführungsform von Fig. 2a, jedoch als räumliche Darstellung mit Blickrichtung schräg von oben auf die Oberseite des Implantats;
- Fign. 3a-c: eine ähnliche Ausführungsform wie Fign. 2a-c, jedoch mit einer Ausführungsform des erfindungsgemäßen Implantats in mittlerer M-Größe;
- Fign. 4a-c: eine ähnliche Ausführungsform wie Fign. 2a-c, jedoch mit einer Ausführungsform des erfindungsgemäßen Implantats in kleiner S-Größe;
- Fig. 5: eine schematische Draufsicht in stark vergrößerter Darstellung senkrecht von oben auf die Zuschnitts-Ebene einer Ausführungsform des erfindungsgemäßen Implantats mit asymmetrischen Seitenabschnitten in großer L-Größe;
- Fig. 6: eine Ausführungsform des erfindungsgemäßen Implantats mit asymmetrischen Seitenabschnitten in mittlerer M-Größe; und
- Fig. 7: eine Ausführungsform des erfindungsgemäßen Implantats mit asymmetrischen Seitenabschnitten in kleiner S-Größe.

Das in sämtlichen Figuren der Zeichnung dargestellte rhinologische **Implantat 10; 20; 30; 40; 50; 60; 70** zur Begradigung der Nasenscheidewand ist auf einer Außenfläche des Septums der menschlichen Nase in der linken und/oder rechten Seite der Nasenhöhle befestigbar. Im implantierten Zustand liegt dann das Implantat 10; 20; 30; 40; 50; 60; 70 auf dieser Außenfläche des Septums vollflächig an. Es ist aus einem flachen, mit mehreren durchgängigen **Perforationen 15** versehenen Zuschnitt aufgebaut.

Die Perforationen 15 können -wie in den Figuren der Zeichnung gezeigtpolyederförmig oder wabenförmig, aber bei nicht eigens dargestellten Ausführungsform auch als kreisrunde Löcher oder auch als Langlöcher ausgebildet sein. Sie helfen, einerseits das Gewicht des Implantats zu verringern und andererseits den Anteil des körperfremden Materials im Körper eines Patienten so weit wie möglich zu reduzieren. Außerdem fördern die Perforationen 15 das Verwachsen des Implantats 10; 20; 30; 40; 50; 60; 70 mit dem umgebenden Gewebe.

Die Perforationen 15 weisen bei den in der Zeichnung dargestellten Ausführungsformen der Erfindung in Summe einen größeren Flächeninhalt auf als die die Perforationen 15 umgebenden, Steg-förmig ausgebildeten, **festen Teilabschnitte 15'** des Implantats 10; 20; 30; 40; 50; 60; 70.

Das Implantat 10; 20; 30; 40; 50; 60; 70 wird operativ durch eine sogenannte offene Rhinoplastik in die menschliche Nase eingebracht und auf dem Knorpel des Septums mittels einer Naht befestigt. Hierbei werden mehrere Einzelnähte durch die Perforationen 15 und das Septum angelegt und fixiert.

Erfindungsgemäß zeichnet sich das Implantat 10; 20; 30; 40; 50; 60; 70 dadurch aus, dass es stets eine ebene Form aufweist und im implantierten Zustand flach auf lediglich einer einzigen der beiden Außenflächen des Septums vollflächig anliegt.

Das erfindungsgemäße Implantat 10; 20; 30; 40; 50; 60; 70 weist einen **dreiecksförmigen Grundkörper 11; 21; 31; 41; 51; 61; 71** auf, bei welchem von einer **Dreiecksspitze** S ausgehend zwei **Katheten-Kanten 12a,13a** unter einem **Dreieckswinkel** φ gegeneinander angeordnet sind, geradlinig von der Dreiecksspitze S wegführen und beiderseits jeweils in Richtung auf eine der Dreiecksspitze S gegenüberliegende **Hypotenusen-Kante 14a** verlaufen.

Der dreiecksförmige Grundkörper 11; 21; 31; 41; 51; 61; 71 ist an den beiden Katheten-Kanten 12a,13a durch zwei jeweils von der Dreiecksspitze S weg über die Hypotenusen-Kante 14a hinausgreifende **Seitenabschnitte 12,13; 22,23; 32,33; 42,43; 52,53; 62,63; 72,73** verlängert, wobei die **Außen-Kanten 12a',13a'** der beiden Seitenabschnitte 12,13; 22,23; 32,33; 42,43; 52,53; 62,63; 72,73 jeweils in Flucht mit ihrer jeweiligen Katheten-Kante 12a,13a verlaufen.

Bei sämtlichen in der Zeichnung dargestellten Ausführungsformen des erfindungsgemäßen Implantats 10; 20; 30; 40; 50; 60; 70 weisen die beiden Seitenabschnitte 12,13; 22,23; 32,33; 42,43; 52,53; 62,63; 72,73 jeweils eine zu ihrer Außen-Kante 12a' bzw. 13a' mit Abstand parallel verlaufende **Innen-Kante 12b' bzw. 13b'** auf.

Die Dicke des flachen Zuschnitts für das Implantat 10; 20; 30; 40 beträgt in der Regel zwischen 0,2mm und 1,2mm, vorzugsweise etwa 0,8mm, und die maximale Längsausdehnung zwischen 2mm und 7mm, vorzugsweise etwa 5mm.

Der Dreieckswinkel φ an der Dreiecksspitze S des dreiecksförmigen Grundkörpers 11; 21; 31; 41; 51; 61; 71 kann zwischen 60° und 120° betragen, in den gezeigten Ausführungsformen liegt er etwa bei 100°.

Bei sämtlichen in der Zeichnung dargestellten Ausführungsformen sind die Dreiecksspitze S sowie auch alle weiteren, zumindest die außenliegenden, Ecken des Implantats 10; 20; 30; 40; 50; 60; 70 abgerundet ausgeführt.

Bei in der Zeichnung nicht dargestellten Ausführungsformen der Erfindung können auch einige oder sämtliche Kanten 12a, 13a, 14a, 12a', 13a', 12b', 13b' des Implantats 10; 20; 30; 40; 50; 60; 70, insbesondere zumindest alle außenliegenden Kanten, abgerundet ausgeführt sein.

Ebenfalls nicht eigens in der Zeichnung dargestellt sind Ausführungsformen der Erfindung, bei welchen in den dreiecksförmigen Grundkörper 11; 21; 31; 41; 51; 61; 71 und/oder in die Seitenabschnitte 12,13; 22,23; 32,33; 42,43; 52,53; 62,63; 72,73 ein oder mehrere Spikes eingearbeitet sind.

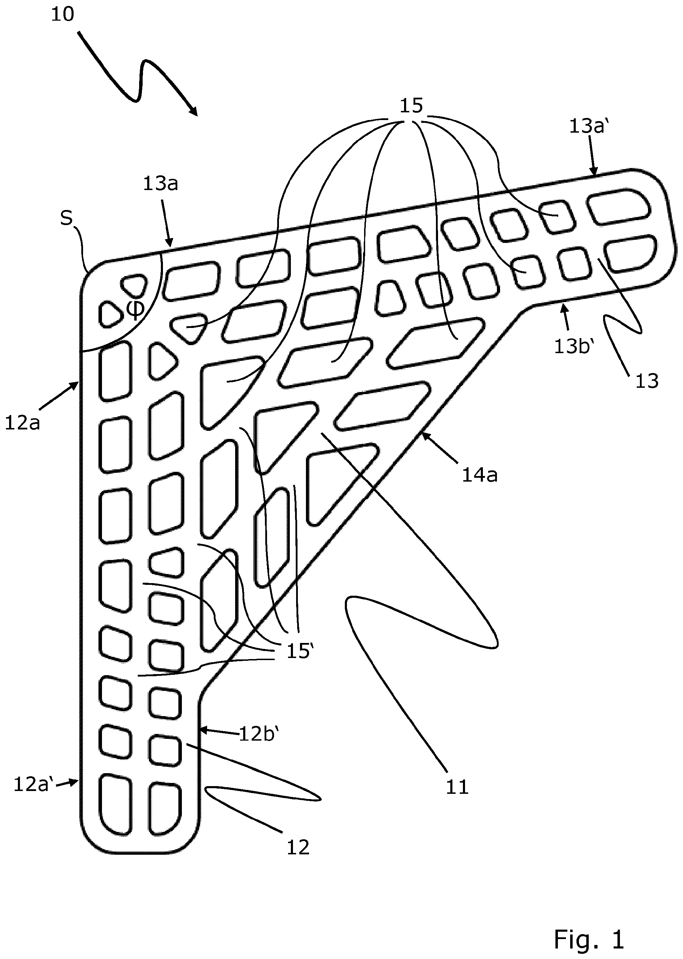

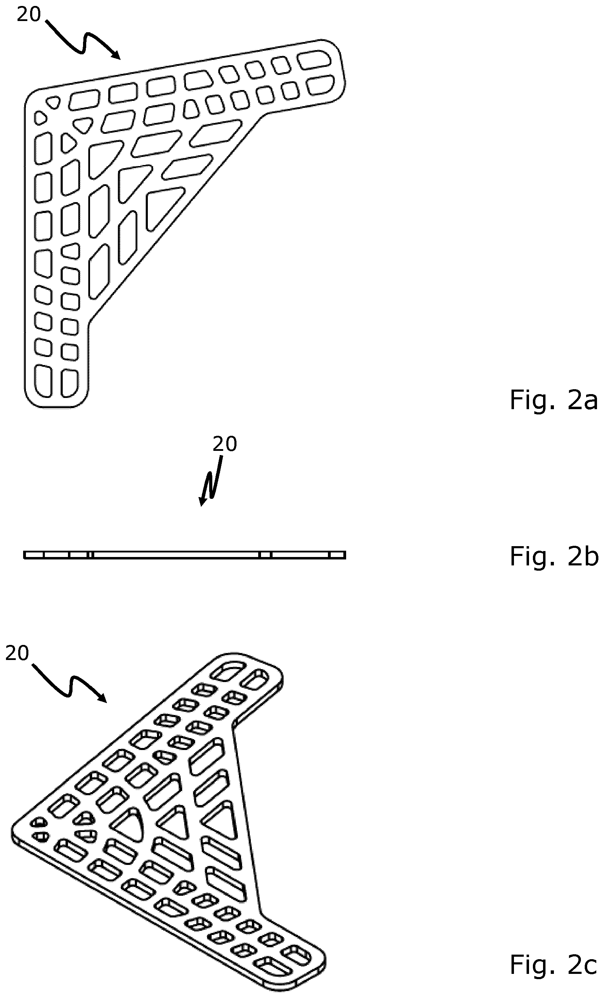

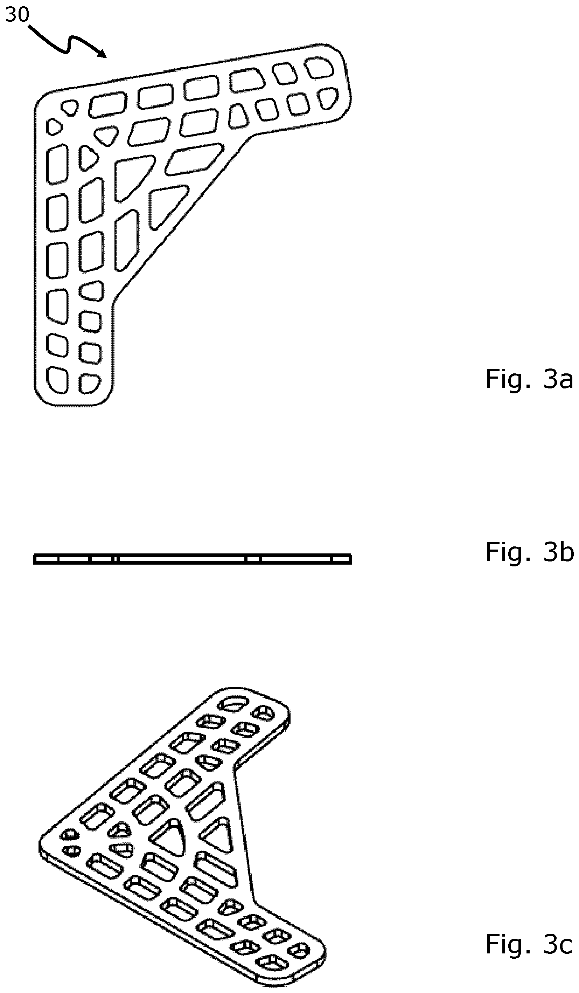

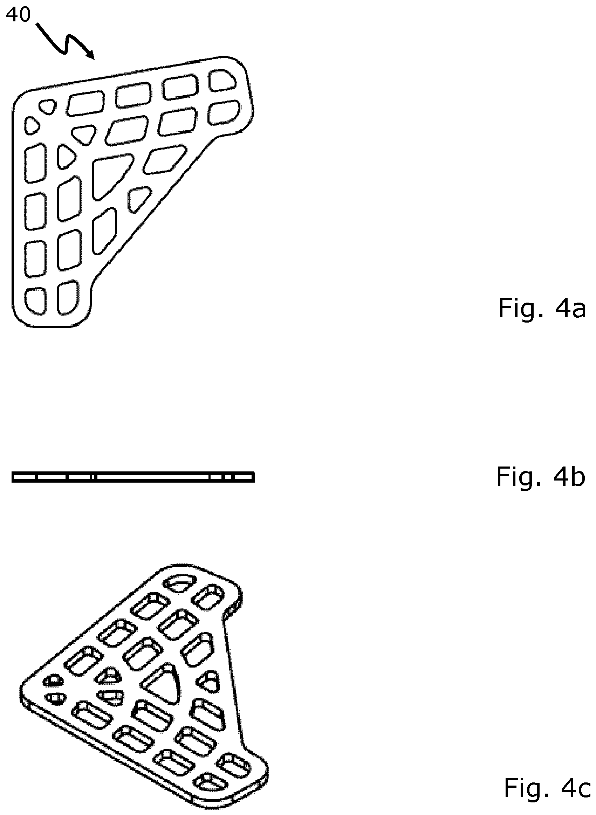

Die **Figuren 1 bis 4c** der Zeichnung stellen Ausführungsformen der Erfindung dar, bei welchen das Implantat 10; 20; 30; 40 symmetrisch zu einer von der Dreiecksspitze S rechtwinklig auf die Hypotenusen-Kante 14a verlaufenden gedachten Höhen-Achse aufgebaut ist, die das Implantat 10; 20; 30; 40 lateral in zwei spiegelbildliche Hälften aufteilt.

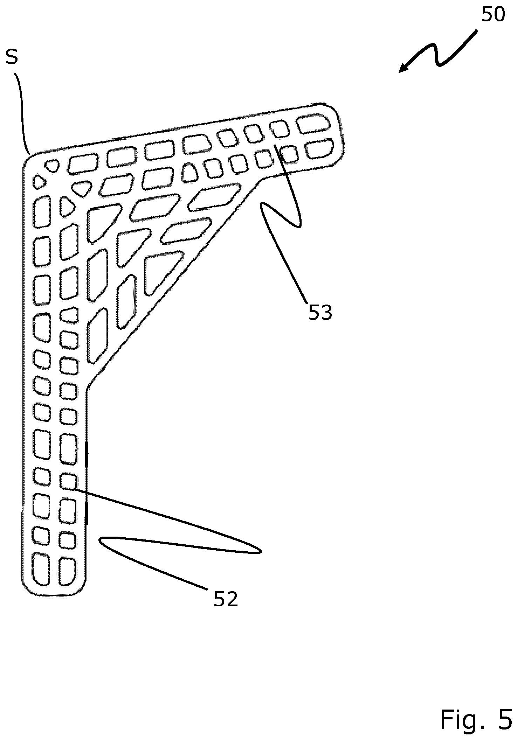

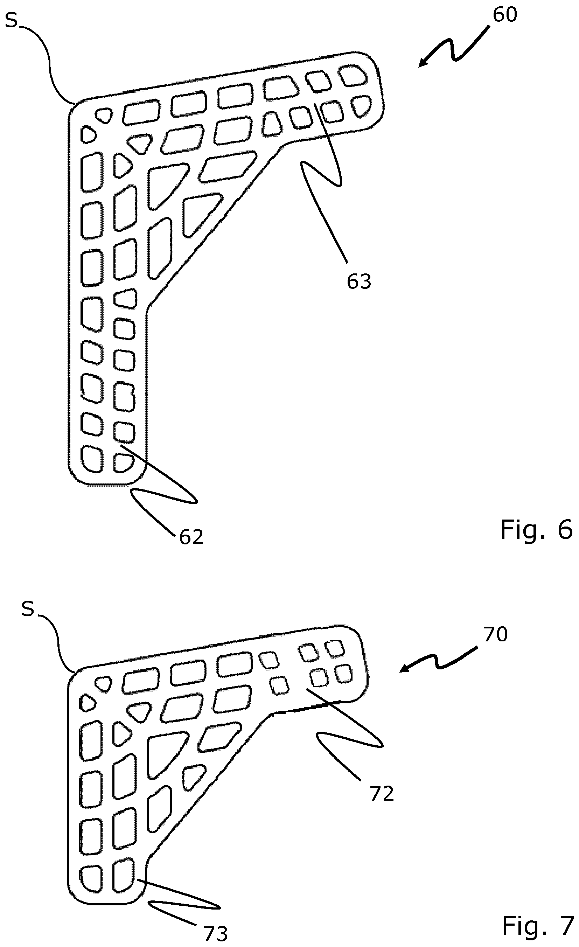

Als Alternativen dazu zeigen die **Figuren 5 bis 7** der Zeichnung Ausführungsformen der Erfindung, bei denen das Implantat 50; 60; 70 asymmetrisch aufgebaut ist, wobei der erste Seitenabschnitt 52; 62; 72 eine größere Längenausdehnung von der Dreiecksspitze S des dreiecksförmigen Grundkörpers 51; 61; 71 weg aufweist als der zweite Seitenabschnitt 53; 63; 73.

Das erfindungsgemäße Implantat 10; 20; 30; 40; 50; 60; 70 kann ganz oder teilweise aus Metall, insbesondere aus Titan, vorzugsweise aus Rein-Titan oder einer Titanlegierung, oder aus Edelstahl und/oder aus einem Werkstoff mit Memory-Effekt und/oder superelastischen Eigenschaften, vorzugsweise aus Nitinol, gefertigt sein. Vorzugsweise wird es einen körperverträglichen, antibakteriellen Überzug aufweisen.

Das Implantat 10; 20; 30; 40; 50; 60; 70 kann aber auch ganz oder teilweise aus Kunststoff gefertigt sein, insbesondere aus Polymer-Material, vorzugsweise aus einem Material, welches eine ähnliche Flexibilität wie das menschliche Septum aufweist, aber über genügend Steifigkeiten verfügt, um eine gekrümmte Nasenscheidewand langfristig zu begradigen, und ohne eine Durchtrennung des Septums erforderlich zu machen, um eine Schwächung herbeizuführen.

Das Implantat 10; 20; 30; 40; 50; 60; 70 kann mittels 3D-Drucktechnik und/oder mittels Lasertechnik hergestellt werden. Möglich ist auch, das Implantat 10; 20; 30; 40; 50; 60; 70 in spanender Technik, insbesondere mittels Frästechnik und/oder mittels Schleiftechnik, vorzugsweise Gleitschleifen, Magnetgleitschleifen oder Satellitenfliehkraftschleifen, und/oder mittels Poliertechnik, vorzugsweise Elektropolieren, oder in Ätztechnik herzustellen und zu bearbeiten. Außerdem kann das Implantat 10; 20; 30; 40; 50; 60; 70 beispielsweise auch im Spritzguss nach dem Micro Injection Moulding (=MIM) Verfahren gefertigt werden.

Hauptzweck des erfindungsgemäßen Implantats ist eine Stabilisierung, Schienung und Begradigung der vorderen (anterioren) Nasenscheidewand bis zum hinteren (dorsalen) Ende des Implantats. Indikationen können zum Beispiel Septums-Deviationen des anterioren Septums bis zu ca. 15mm in Folge von Traumata, kongenitaler Deviation usw. sein.

### Bezugszeichenliste:

- 10; 20; 30; 40; 50; 60; 70: rhinologisches Implantat
- 11; 21; 31; 41; 51; 61; 71: dreiecksförmiger Grundkörper
- 12,13; 22,23; 32,33; 42,43; 52,53; 62,63; 72,73: hinausgreifende Seitenabschnitte
- 12a, 13a: Katheten-Kanten
- 14a: Hypotenusen-Kante
- 12a', 13a': Außen-Kanten der Seitenabschnitte
- 12b', 13b': Innen-Kanten der Seitenabschnitte
- 15: Perforationen
- 15': feste Teilabschnitte
- S: Dreiecksspitze
- φ: Dreieckswinkel

### Referenzliste:

Für die Beurteilung der Patentfähigkeit in Betracht gezogene Publikationen:
[1] DE 10 2022 120 193 B3 ≈ EP 23 180 536.7
[2] DE 10 2012 107 123 B4 ≈ EP 2 692 313 B1 ≈ US 9 895 252 B2
[3] Internetseite vom 19.01.2024
   https://bess.eu/rhinologie/septumschienen/septumschienen
[4] US 2012/0078367 A1
[5] WO 2008/153263 A1
[6] US 6 322 590 B1
[7] EP 1 475 056 B1
[8] DE 10 2006 023 058 B3
[9] HNO. 1999 Jun; 47(6):546-50
[10] CN 21 538 4901 U
[11] US 11 241 306 B2
[12] WO 00/06327 A2

## Patentansprüche

1. Rhinologisches Implantat (10; 20; 30; 40; 50; 60; 70) zur Begradigung der Nasenscheidewand, das auf einer Außenfläche des Septums der menschlichen Nase in der linken und/oder rechten Seite der Nasenhöhle befestigbar ist und im implantierten Zustand auf dieser Außenfläche des Septums vollflächig anliegt, wobei das Implantat (10; 20; 30; 40; 50; 60; 70) aus einem flachen, mit mehreren durchgängigen Perforationen (15) versehenen Zuschnitt aufgebaut ist,
**dadurch gekennzeichnet,**
**dass** das Implantat (10; 20; 30; 40; 50; 60; 70) eine ebene Form aufweist und im implantierten Zustand flach auf lediglich einer einzigen der beiden Außenflächen des Septums vollflächig anliegt; dass das Implantat (10; 20; 30; 40; 50; 60; 70) einen dreiecksförmigen Grundkörper (11; 21; 31; 41; 51; 61; 71) aufweist, bei welchem von einer Dreiecksspitze (S) ausgehend zwei Katheten-Kanten (12a,13a) unter einem Dreieckswinkel (φ) gegeneinander angeordnet sind, geradlinig von der Dreiecksspitze (S) wegführen und beiderseits jeweils in Richtung auf eine der Dreiecksspitze (S) gegenüberliegende Hypotenusen-Kante (14a) verlaufen;
und **dass** der dreiecksförmige Grundkörper (11; 21; 31; 41; 51; 61; 71) an den beiden Katheten-Kanten (12a,13a) durch zwei jeweils von der Dreiecksspitze (S) weg über die Hypotenusen-Kante (14a) hinausgreifende Seitenabschnitte (12,13; 22,23; 32,33; 42,43; 52,53; 62,63; 72,73) verlängert ist, wobei die Außen-Kanten (12a',13a') der beiden Seitenabschnitte (12,13; 22,23; 32,33; 42,43; 52,53; 62,63; 72,73) jeweils in Flucht mit ihrer jeweiligen Katheten-Kante (12a,13a) verlaufen.

2. Implantat nach Anspruch 1, **dadurch gekennzeichnet, dass** die Dreiecksspitze (S) sowie vorzugsweise auch alle weiteren, zumindest die außenliegenden Ecken des Implantats (10; 20; 30; 40; 50; 60; 70) abgerundet ausgeführt sind.

3. Implantat nach Anspruch 1 oder 2, **dadurch gekennzeichnet, dass** die beiden Seitenabschnitte (12,13; 22,23; 32,33; 42,43; 52,53; 62,63; 72,73) jeweils eine zu ihrer Außen-Kante (12a' bzw. 13a') mit Abstand parallel verlaufende Innen-Kante (12b' bzw. 13b') aufweisen.

4. Implantat nach einem der Ansprüche 1 bis 3, **dadurch gekennzeichnet, dass** das Implantat (10; 20; 30; 40) symmetrisch zu einer von der Dreiecksspitze (S) rechtwinklig auf die Hypotenusen-Kante (14a) verlaufenden Höhen-Achse, die das Implantat (10; 20; 30; 40) lateral in zwei spiegelbildliche Hälften aufteilt, aufgebaut ist.

5. Implantat nach einem der Ansprüche 1 bis 3, **dadurch gekennzeichnet, dass** das Implantat (50; 60; 70) asymmetrisch aufgebaut ist, wobei ein erster Seitenabschnitt (52; 62; 72) eine größere Längenausdehnung von der Dreiecksspitze (S) des dreiecksförmigen Grundkörpers (51; 61; 71) weg aufweist als der zweite Seitenabschnitt (53; 63; 73).

6. Implantat nach einem der vorhergehenden Ansprüche, **dadurch gekennzeichnet, dass** in den dreiecksförmigen Grundkörper (11; 21; 31; 41; 51; 61; 71) und/oder in die Seitenabschnitte (12,13; 22,23; 32,33; 42,43; 52,53; 62,63; 72,73) ein oder mehrere Spikes eingearbeitet sind.

7. Implantat nach einem der vorhergehenden Ansprüche, **dadurch gekennzeichnet, dass** die Perforationen (15) zusammen einen größeren Flächeninhalt aufweisen als die die Perforationen (15) umgebenden, insbesondere stegförmig ausgebildeten, festen Teilabschnitte (15') des Implantats (10; 20; 30; 40; 50; 60; 70), und dass vorzugsweise zumindest ein Teil der Perforationen (15) polyederförmig oder wabenförmig ausgebildet ist.

8. Implantat nach einem der vorhergehenden Ansprüche, **dadurch gekennzeichnet, dass** sämtliche Kanten (12a, 13a, 14a, 12a', 13a, 12b', 13b') des Implantats (10; 20; 30; 40; 50; 60; 70), zumindest alle außenliegenden Kanten, abgerundet ausgeführt sind.

9. Implantat nach einem der vorhergehenden Ansprüche, **dadurch gekennzeichnet, dass** die Dicke des flachen Zuschnitts für das Implantat (10; 20; 30; 40) zwischen 0,2mm und 1,2mm, vorzugsweise etwa 0,8mm, und die maximale Längsausdehnung zwischen 2mm und 7mm, vorzugsweise etwa 5mm, beträgt.

10. Implantat nach einem der vorhergehenden Ansprüche, **dadurch gekennzeichnet, dass** der Dreieckswinkel (φ) an der Dreiecksspitze (S) des dreiecksförmigen Grundkörpers (11; 21; 31; 41; 51; 61; 71) zwischen 60° und 120°, vorzugsweise etwa 100°, beträgt.

11. Implantat nach einem der vorhergehenden Ansprüche, **dadurch gekennzeichnet, dass** das Implantat (10; 20; 30; 40; 50; 60; 70) einen körperverträglichen Überzug aufweist.

12. Implantat nach einem der Ansprüche 1 bis 11, **dadurch gekennzeichnet, dass** das Implantat (10; 20; 30; 40; 50; 60; 70) aus Metallblech, insbesondere aus reinem Titan oder einer Titanlegierung, oder aus einem Werkstoff mit Memory-Effekt und/oder superelastischen Eigenschaften, vorzugsweise aus Nitinol, aufgebaut ist.

13. Implantat nach einem der Ansprüche 1 bis 11, **dadurch gekennzeichnet, dass** das Implantat (10; 20; 30; 40; 50; 60; 70) ganz oder teilweise aus Kunststoff gefertigt ist, insbesondere aus Polymer-Material, vorzugsweise aus einem Material, welches eine ähnliche Flexibilität wie das menschliche Septum aufweist, aber über genügend Steifigkeiten verfügt, um eine gekrümmte Nasenscheidewand langfristig zu begradigen, und ohne eine Durchtrennung des Septums erforderlich zu machen um eine Schwächung herbeizuführen.

## Claims

1. A rhinological implant (10; 20; 30; 40; 50; 60; 70) for straightening the nasal septum, which can be fixed an outer surface of the septum of the human nose on the left and/or on the right side of the nasal cavity and, when implanted, lies flat against this outer surface of the septum, wherein the implant (10; 20; 30; 40; 50; 60; 70) is constructed from a flat blank provided with a plurality of continuous perforations (15),
**characterized in**
**that** the implant (10; 20; 30; 40; 50; 60; 70) has a planar shape and, when implanted, lies flat against only one of the two outer surfaces of the septum over its entire surface;
**that** the implant (10; 20; 30; 40; 50; 60; 70) has a triangular base body (11; 21; 31; 41; 51; 61; 71) in which two cathetus edges (12a, 13a) are arranged extending straight away from a triangle apex (S) at a triangular angle (φ) relative to each other, extend in a straight line away from the apex (S) of the triangle and run on both sides toward a hypotenuse edge (14a) opposite the apex (S) of the triangle;
and **that** the triangular base body (11; 21; 31; 41; 51; 61; 71) is extended at the two cathetus edges (12a, 13a) by two side sections (12,13; 22,23; 32,33; 42,43; 52,53; 62,63; 72,73) extending beyond the hypotenuse edge (14a) away from the triangle apex (S), wherein the outer edges (12a', 13a') of the two side sections (12,13; 22,23; 32,33; 42,43; 52,53; 62,63; 72,73) each run in alignment with their respective cathetus edge (12a, 13a).

2. The implant according to claim 1, **characterized in that** the triangle apex (S) and preferably also all other corners of the implant (10; 20; 30; 40; 50; 60; 70), at least the outer corners, are rounded.

3. The implant according to claim 1 or 2, **characterized in that** the two side sections (12,13; 22,23; 32,33; 42,43; 52,53; 62,63; 72,73) each have an inner edge (12b' or 13b') that runs parallel to and at a distance from its outer edge (12a' or 13a').

4. The implant according to anyone of claims 1 to 3, **characterized in that** the implant (10; 20; 30; 40) is constructed symmetrically with respect to a height axis extending at right angles from the triangle apex (S) of the triangle to the hypotenuse edge (14a), which divides the implant (10; 20; 30; 40) laterally into two mirror-image halves.

5. The implant according to anyone of claims 1 to 3, **characterized in that** the implant (50; 60; 70) is constructed asymmetrically, wherein a first side section (52; 62; 72) has a greater length extending away from the triangle apex (S) of the triangular base body (51; 61; 71) than the second side section (53; 63; 73).

6. The implant according to anyone of the preceding claims, **characterized in that** one or more spikes are incorporated into the triangular base body (11; 21; 31; 41; 51; 61; 71) and/or into the side sections (12,13; 22,23; 32,33; 42,43; 52,53; 62,63; 72,73).

7. The implant according to anyone of the preceding claims, **characterized in that** the perforations (15) together have a larger surface area than the solid sections (15') of the implant (10; 20; 30; 40; 50; 60; 70) surrounding the perforations (15), and that preferably at least some of the perforations (15) are polyhedral or honeycomb-shaped.

8. The implant according to anyone of the preceding claims, **characterized in that** all edges (12a, 13a, 14a, 12a', 13a', 12b', 13b') of the implant (10; 20; 30; 40; 50; 60; 70), at least all outer edges, are rounded.

9. The implant according to anyone of the preceding claims, **characterized in that** the thickness of the flat blank for the implant (10; 20; 30; 40) is between 0.2 mm and 1.2 mm, preferably about 0.8 mm, and the maximum longitudinal extension is between 2 mm and 7 mm, preferably about 5 mm.

10. The implant according to anyone of the preceding claims, **characterized in that** the triangle angle (φ) at the triangle apex (S) of the triangular base body (11; 21; 31; 41; 51; 61; 71) is between 60° and 120°, preferably about 100°.

11. The implant according to anyone of the preceding claims, **characterized in that** the implant (10; 20; 30; 40; 50; 60; 70) has a biocompatible coating.

12. The implant according to anyone of claims 1 to 11, **characterized in that** the implant (10; 20; 30; 40; 50; 60; 70) is made of sheet metal, in particular pure titanium or a titanium alloy, or a material with a memory effect and/or superelastic properties, preferably Nitinol.

13. The implant according to anyone of claims 1 to 11, **characterized in that** the implant (10; 20; 30; 40; 50; 60; 70) is made entirely or partially of plastic, in particular of polymer material, preferably of a material which has a flexibility similar to that of the human septum, but has sufficient rigidity to straighten a curved nasal septum in the long term without requiring the septum to be cut in order to weaken it.

## Revendications

1. Implant rhinologique (10; 20; 30; 40; 50; 60; 70) destiné à redresser la cloison nasale, qui peut être fixé sur une surface extérieure du septum du nez humain, dans la partie gauche et/ou droite de la cavité nasale, et qui, à l'état implanté, repose sur toute sa surface contre cette surface extérieure du septum, l'implant (10; 20; 30; 40; 50; 60; 70) est constitué d'une découpe plate pourvue de plusieurs perforations traversantes (15),
**caractérisé en ce que**
l'implant (10; 20; 30; 40; 50; 60; 70) présente une forme plane et, à l'état implanté, repose à plat sur une seule des deux surfaces extérieures du septum sur toute sa surface;
que l'implant (10; 20; 30; 40; 50; 60; 70) présente un corps de base triangulaire (11; 21; 31; 41; 51; 61; 71) dans lequel, à partir d'une pointe du triangle (S) du triangle, deux côtés cathètes (12a, 13a) sont disposés l'un par rapport à l'autre sous un angle triangulaire (φ), s'éloignent en ligne droite de la pointe du triangle (S) et s'étendent de chaque côté vers un côté hypoténuse (14a) opposé à la pointe du triangle (S);
et que le corps de base triangulaire (11; 21; 31; 41; 51; 61; 71) est prolongé au niveau des deux côtés cathètes (12a, 13a) par deux sections latérales (12,13; 22,23; 32,33; 42,43; 52,53; 62,63; 72,73) s'étendant respectivement au-delà de la pointe du triangle (S) au-delà du côté hypotenuse (14a), les bords extérieurs (12a', 13a') des deux sections latérales (12,13; 22,23; 32,33; 42,43; 52,53; 62,63; 72,73) s'étendent respectivement dans l'alignement de leur côtés cathètes (12a, 13a).

2. Implant selon la revendication 1, **caractérisé en ce que** la pointe du triangle (S) ainsi que, de préférence, tous les autres angles, au moins les angles extérieurs de l'implant (10; 20; 30; 40; 50; 60; 70) sont arrondis.

3. Implant selon la revendication 1 ou 2, **caractérisé en ce que** les deux sections latérales (12,13; 22,23; 32,33; 42,43; 52,53; 62,63; 72, 73) présentent chacune un bord intérieur (12b' ou 13b') parallèle à leur bord extérieur (12a' ou 13a') à une certaine distance.

4. Implant selon l'une des revendications 1 à 3, **caractérisé en ce que** l'implant (10; 20; 30; 40) est construit symétriquement par rapport à un axe de hauteur s'étendant perpendiculairement à partir de la pointe du triangle (S) vers le bord de l'hypoténuse (14a), qui divise latéralement l'implant (10; 20; 30; 40) en deux moitiés symétriques.

5. Implant selon l'une des revendications 1 à 3, **caractérisé en ce que** l'implant (50; 60; 70) est de conception asymétrique, une première partie latérale (52; 62; 72) présentant une plus grande longueur à partir de la pointe du triangle (S) du corps de base triangulaire (51; 61; 71) que la deuxième partie latérale (53; 63; 73).

6. Implant selon l'une des revendications précédentes, **caractérisé en ce qu'**un ou plusieurs picots sont intégrés dans le corps de base triangulaire (11; 21; 31; 41; 51; 61; 71) et/ou dans les sections latérales (12,13; 22,23; 32,33; 42,43; 52,53; 62,63; 72,73).

7. Implant selon l'une des revendications précédentes, **caractérisé en ce que** les perforations (15) présentent ensemble une surface plus grande que les parties solides (15') de l'implant (10; 20; 30; 40; 50; 60; 70) qui entourent les perforations (15), et **en ce que**, de préférence, au moins une partie des perforations (15) est en forme de polyèdre ou de nid d'abeilles.

8. Implant selon l'une des revendications précédentes, **caractérisé en ce que** tous les bords (12a, 13a, 14a, 12a', 13a', 12b', 13b') de l'implant (10; 20; 30; 40; 50; 60; 70), au moins tous les bords extérieurs, sont arrondis.

9. Implant selon l'une des revendications précédentes, **caractérisé en ce que** l'épaisseur de la découpe plate pour l'implant (10; 20; 30; 40) est comprise entre 0,2 mm et 1,2 mm, de préférence d'environ 0,8 mm, et que la longueur maximale est comprise entre 2 mm et 7 mm, de préférence environ 5 mm.

10. Implant selon l'une des revendications précédentes, **caractérisé en ce que** l'angle triangulaire (φ) à la pointe du triangle (S) du corps de base triangulaire (11; 21; 31; 41; 51; 61; 71) est comprise entre 60° et 120°, de préférence d'environ 100°.

11. Implant selon l'une des revendications précédentes, **caractérisé en ce que** l'implant (10; 20; 30; 40; 50; 60; 70) présente un revêtement biocompatible.

12. Implant selon l'une des revendications 1 à 11, **caractérisé en ce que** l'implant (10; 20; 30; 40; 50; 60; 70) est constitué de tôle métallique, en particulier de titane pur ou d'un alliage de titane, ou d'un matériau à effet mémoire et/ou à propriétés superélastiques, de préférence en nitinol.

13. Implant selon l'une des revendications 1 à 11, **caractérisé en ce que** l'implant (10; 20; 30; 40; 50; 60; 70) est fabriqué entièrement ou partiellement en matière plastique, en particulier en polymère, de préférence en un matériau qui présente une flexibilité similaire à celle du septum humain, mais qui dispose d'une rigidité suffisante pour redresser à long terme une cloison nasale courbée, sans nécessiter de sectionnement du septum pour provoquer un affaiblissement.
